# EUROPEAN PATENT APPLICATION

(11) **EP 2 272 831 A1**
(43) Date of publication of application: **12.01.2011**
(21) Application number: 09163878.3
(22) Date of filing: 26.06.2009
(51) Int. Cl.: C07D 213/46

(54) **Process for the preparation of arylpyridinyl compounds**

(71) Applicant: Prime European Therapeuticals S.p.A., 20121 Milano (IT)
(72) Inventor: Scrocchi, Roberto, 27100, PAVIA (IT); Fedeli, Palma, 26856, SENNA LODIGIANA (LODI) (IT); Argese, Maria, 20018, SEDRIANO (MI) (IT); Guazzi, Giuseppe, 20133, MILANO (IT)
(74) Representative: Minoja, Fabrizio

(57) **Abstract**

A process is described for the preparation of arylpyridine compounds by aryl-aryl cross-coupling reactions between a halopyridine and an arylmagnesium halide carried out in the presence of a catalytic amount of a zinc salt and a catalytic amount of palladium complex with a bidentate phosphine. The zinc salt is preferably selected from ZnCl₂, ZnBr₂ and/or Zn(OAc)2_{,} while the palladium complex with a bidentate phosphine is preferably selected from the group of (1,2-Bis(diphenylphosphino)ethane)palladium(II) chloride, (1,3-Bis(diphenylphosphino)propane) palladium(II) chloride and (1,4-Bis(diphenylphosphino)butane)palladium(II) chloride. Most preferred is (1,2-Bis(diphenylphosphino)ethane)palladium(II) chloride. It is thus possible to obtain molar yields higher than 95% calculated on the arylmagnesium halide and a catalyticity less than 1:1500. The process is particularly suitable for the preparation of 4-(2'-pyridyl)benzaldehyde which can then effectively been converted to Nl-(t-butoxycarbonyl)-N2-(4-(2'pyridyl)benzyl)hydrazine.

## Description

Arylpyridines are generally used in organic synthesis as intermediates for the preparation of various kinds of compound; of these, 4-(2'-pyridyl)benzaldehyde is a useful intermediate in the preparation of antiviral drugs and, in particular, of HIV protease inhibitors, such as, for example, the azahexane heterocyclic derivatives described in international patent application WO 97/40029, which is incorporated herein by reference; among the antiviral drugs concerned, one of particular interest is, for example, that indicated by the abbreviation BMS-232632 in Drugs of the Future 1999, 24(4):375, the structural formula of which is given below.

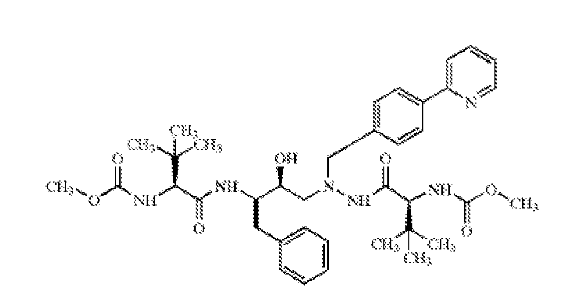

US 6,765,097 B1 discloses a process for the preparation of arylpyridine compounds comprising reacting an halopyridine and an arylmagnesium halide (Grignard's reagent) in the presence of catalytic amounts of a zinc salt and palladium. The zinc salt is generally selected from ZnCl₂, ZnBr₂ and Zn(OAc)₂, while the palladium is used principally in the form of palladium tetrakistriphenylphosphine [Pd(PPh₃)₄ ] or palladium salts, generally acetate or chloride. Bidentate phosphines such as 1,3-bis(diphenylphosphine)propane (DPPP) or 1,4-is(diphenylphosphine)butane (DPPB) may optionally be present.

In particular, US 6,765,097 B1 discloses cross-coupling reactions suitable for the preparation of 4-(2'-pyridyl)benzaldehyde, which are performed according to the following Scheme 1. 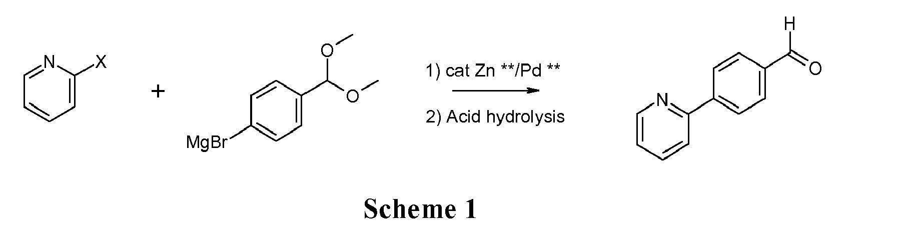 wherein X is Br or Cl. and cat Zn**/Pd** represents the catalytic system comprising the above mentioned zinc salts, palladium salts or complexes, and phosphines.

The catalytic system is different depending on the nature of X. The following table summarizes some results disclosed in US 6,765,097 B1 when X is Cl.

| **Example #** | **Molar Ratio 2-Chloropyridine/ Grignard's Reagent** | **Pd Catalyst** | **% Yield Based on 2-Chloropyridine** | **% Yield Based on Grignard's Reagent** |
|---|---|---|---|---|
| 17 | 0.736 | Pd(PPh₃)₄ | 84.0 | 61.8 |
| 18 | 0.881 | Pd(OAc)₂/DPPP | 97.0 | 84.5 |
| 19 | 0.881 | Pd(OAc)₂/DPPP | 97.8 | 86.2 |
| 20 | 0.881 | Pd(OAc)₂/DPPP | 99.4 | 87.6 |
| 21 | 0.895 | Pd(OAc)₂/DPPB | 100,0 | 89.5 |
| 23 | 0.900 | Pd(OAc)₂/DPPP | 95.0 | 85.5 |

During the scale up of the process for the preparation of 4-(2'-pyridyl)benzaldehyde according to Example 23 of US 6,765,097 B1, a yield of approximately 90% based on 2-chloropyridine and of approximately 80% based on the Grignard's reagent was unexpectedly obtained. Problems due the presence of insoluble materials were experienced during the aqueous work-up, which affected the isolation of the toluene solution of the aldehyde as well as the subsequent steps to produce N1-(tert-butoxycarbonyl)-N2-[4-(2'-pyridyl)benzyl]hydrazine. Specifically, reaction of the toluene solution of the aldehyde with tert-butyl carbazate provided N1-(tert-butoxycarbonyl)-N2-[4-[(2-pyridylphenyl)]methylidene] hydrazone with an overall yield of 77.8% based on 4-bromobenzaldehyde dimethyl acetal, while the yield of the final reduction step was only 76%. The low yield in the final step was due to the slow reaction rate and to the high content of the by-product 4-(2'-pyridyl)toluene. The slow reaction rate was due to the impurities coming from the coupling step that inhibit the catalytic hydrogenation.

It has now surprisingly been found that when the cross-coupling reaction between a halopyridine and an arylmagnesium halide (Grignard's reagent) is performed in the presence of catalytic amounts of a zinc salt and of a palladium complex with a bidentate phosphine, the yield of the obtained arylpyridine based on the arylmagnesium halide is significantly increased and it is typically higher than 95%.

In addition, when such cross-coupling reaction is applied in a multistep process for the preparation of N1-(tert-butoxycarbonyl)-N2-[4-(2'-pyridyl)benzyl]hydrazine comprising;
a) the preparation of 4-(2'-pyridyl)benzaldehyde through a cross-coupling reaction between a halopyridine and an arylmagnesium halide in the presence of catalytic amounts of a zinc salt and of a palladium complex with a bidentate phosphine according to this invention;
b) conversion of the latter into N1-(tert-butoxycarbonyl)-N2-[4-[(2-pyridylphenyl)]methylidene] hydrazone; and
c) reduction of the hydrazone to N1-(tert-butoxycarbonyl)-N2-[4-(2'-pyridyl)benzyl]hydrazine
both the hydrazone formation and its reduction proceed in higher yields for the reagent which has the higher molar cost (i.e. the arylmagnesium halide) than applying the cross coupling conditions disclosed in US 6,765,097 B1. In addition the isolation of the hydrazone is easily accomplished, its reduction is faster and the final product N1-(tert-butoxycarbonyl)-N2-[4-(2'-pyridyl)benzyl] hydrazine is obtained with a better quality.

It is therefore an object of the present invention a process for the preparation of arylpyridines in which an arylmagnesium halide is reacted with a halopyridine in the presence of a catalytic amount of a zinc salt and a catalytic amount of palladium complex with a bidentate phosphine, wherein the molar ratio of said palladium complex to the halopyridine is less than 1:100 and, normally, less than 1:1000.

In order to avoid any undesired secondary reactions, the arylmagnesium halide and the halopyridine should not contain other substituents capable of interfering with the Grignard reaction or, if such substituents are present, they should be in a suitably protected form; any carbonyl groups can be protected, for example, by being converted beforehand into the corresponding acetals. Accordingly, one object of the present invention is a process represented in the following Scheme 2: 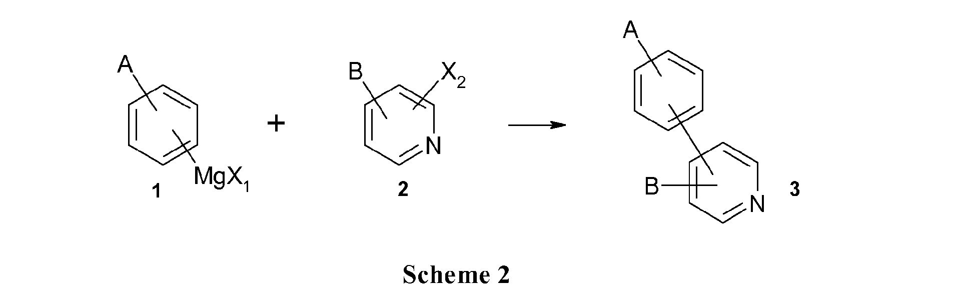 wherein A and B, which are the same or different from one another, represent H; a linear or branched C1 -C8 alkyl; an optionally substituted acetal group; an aryl or a benzyl, which are optionally substituted by groups that do not interfere with a Grignard reaction; X1 and X2, which are the same or different from one another, represent Cl, Br or I; and wherein the reaction between compound 1 and compound 2 to give compound 3 is performed in the presence of catalytic amounts of a zinc salt and of a palladium complex with a bidentate phosphine.

In its preferred embodiment, the process according to the present invention can he represented in the following Scheme 3: 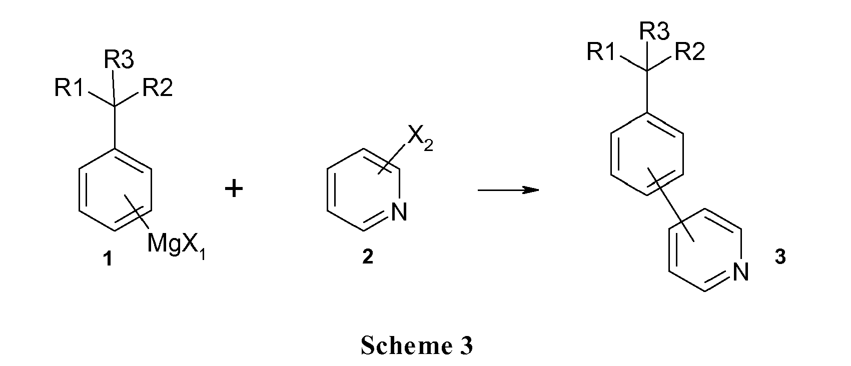 wherein: R1, R2 and R3, which are the same or different from one another, represent H; a linear or branched C1-C6 alkyl; an aryl, preferably phenyl, optionally substituted by a linear or branched C1-C6 alkyl; or, alternatively, R1 and R2 taken together with the carbon atom to which they are attached represent an optionally cyclic acetal group; and X1 and X2, which are the same or different from one another, represent Cl, Br or I.; and wherein the reaction between compound 1 and compound 2 to give compound 3 is performed in the presence of catalytic amounts of a zinc salt and of a palladium complex with a bidentate phosphine.

In its more preferred embodiment, the process consists (a) in reacting an arylmagnesium halide of formula 1: 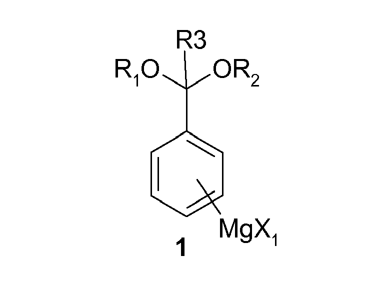 wherein X1 represents Cl, Br or I; R1 and R2, which are the same or different from one another, represent linear or branched C1-C6 alkyls, preferably methyls, or alternatively, R1 and R2 together represent a single C1-C8 alkyl or alkylene group, preferably 1,3-propyl, 1,2-butyl, 1,4-butenyl and 2,2-dimethyl-1,3-propyl; R3 represents hydrogen or a linear or branched C1-C6 alkyl or alkylene radical, with a halopyridine of formula 2: 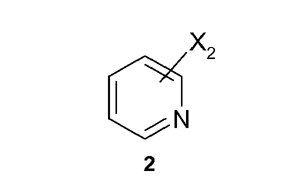 wherein X2 represents Cl, Br or I, in the presence of a catalytic amount of a palladium complex with a bidentate phosphine and of a catalytic amount of a zinc salt, relative to which compound 1 is preferably used in dynamic deficiency, and wherein the molar ratio of the palladium complex with a bidentate phosphine to the arylpyridine product is less than 1:100 and, preferably, less than 1:1000; and (b) in transforming the intermediate compound so obtained into the desired compound by converting the acetal group into a carbonyl group. In particular, it is represented by a process for the preparation of 4-(2'-pyridyl)benzaldehyde in which: (a) an arylmagnesium halide of formula 1 bis: 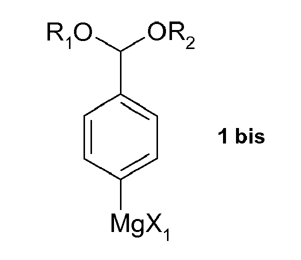 wherein X1, R1 and R2 have the meaning given above, is reacted with a halopyridine of formula 2 bis: 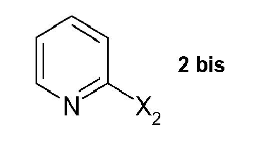 wherein X2 has the meaning given above, in the presence of a catalytic amount of a palladium complex with a bidentate phosphine and of a catalytic amount of a zinc salt, relative to which compound 1 is used in dynamic deficiency; and (b) the intermediate compound so obtained of formula 3 bis: 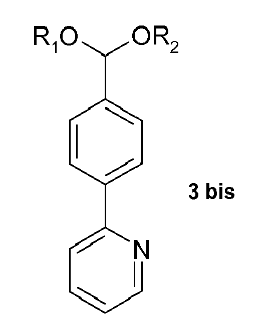 is transformed into 4-(2'-pyridyl)benzaldehyde by converting the acetal group into a carbonyl group.

For the purposes of the present invention, the expression "catalytic amount" of the zinc salt means from 1 to 50 moles of zinc, preferably from 4 to 35 moles, per 100 moles of halopyridine; the expression "catalytic amount" of a palladium complex with a bidentate phosphine, however, means from 0.01 to 1 mole of palladium complex with a bidentate phosphine, preferably from 0.05 to 0.1 mole, per 100 moles of halopyridine; the expression "the Grignard compound is used in dynamic deficiency relative to the zinc salt" means that the arylmagnesium halide is added dropwise to a solution already containing the halopyridine, the palladium complex with a bidentate phosphine and the zinc salt. Finally, the term "catalyticity" means the molar ratio of the catalyst to the halopyridine; owing to the fact that the process according to present invention results in an almost quantitative conversion of the halopyridine into the arylpyridine product, the "catalyticity" in practice coincides with the molar ratio of the catalyst to the arylpyridine product.

Both in its general version and in its preferred version or in its more preferred version, the molar ratio of the palladium complex with a bidentate phosphine to the halopyridine is normally from 1:3000 to 1:1000, preferably approximately 1:2000; the halopyridine is normally used in amounts of from 0.5 to 1.5 moles, preferably from 0.8 to 1.2 moles, per mole of arylmagnesium halide. In a particularly preferred embodiment the molar ratio of the halopyridine to the aryl magnesium halide is 1:1.

In order for the coupling reaction to take place with high yields and a high degree of selectivity in the presence of a minimum amount of catalyst, the Grignard reagent must be prevented from accumulating in the reaction medium, and must thus be in dynamic deficiency relative to the zinc salt; the amount of co-catalyst (Zn salts) necessary depends on the regularity and the speed of addition of the Grignard compound: a ratio of from 1:50 to 1:10 of the Zn salts to the halopyridine has been found to be satisfactory.

The zinc salt is generally selected from zinc chloride (ZnCl₂), zinc bromide (ZnBr₂) and zinc acetate [Zn(OAc)₂ ].

The palladium complex with a bidentate phosphine is preferably selected from the group of (1,2-Bis(diphenylphosphino)ethane)palladium(II) chloride, (1,3-Bis(diphenylphosphino)propane) palladium(II) chloride and (1,4-Bis(diphenylphosphino)butane)palladium(II) chloride. Most preferred is (1,2-Bis(diphenylphosphino)ethane)palladium(II) chloride.

The use of these complexes in combination with the zinc salt makes it possible to obtain molar yields higher than 95% calculated on the arylmagnesium halide and a catalyticity less than 1:1500, using both bromopyridines and the more economical and normally less reactive chloropyridines.

(1,2-Bis(diphenylphosphino)ethane)palladium(II) chloride, (1,3-Bis(diphenylphosphino)propane) palladium(II) chloride and (1,4-Bis(diphenylphosphino)butane)palladium(II) chloride are known, commercially available compounds.

The coupling reaction is generally carried out at a temperature of 25-85°C, preferably at 25-50°C, in an aprotic organic solvent that does not react with a Grignard compound, preferably in tetrahydrofuran and/or toluene.

In the more preferred embodiment of the invention, the removal of the acetal group is effected by acid hydrolysis; that is to say, stage (b) is normally carried out by treating the intermediate (for example 3 bis) with an acidic aqueous solution; this stage is preferably carried out by adding an aqueous HCl solution directly to the organic solution obtained in stage (a) and by maintaining the temperature below 40°C.

Another object of the present invention is a process for the preparation of N1-(tert-butoxycarbonyl)-N2-[4-(2'-pyridyl)benzyl]hydrazine comprising the following steps:
a) providing 4-(2'-pyridyl)benzaldehyde;
b) converting 4-(2'-pyridyl)benzaldehyde into N1-(tert-butoxycarbonyl)-N2-[4-[(2-pyridylphenyl)]methylidene] hydrazone; and
c) reducing N1-(tert-butoxycarbonyl)-N2-[4-[(2-pyridylphenyl)]methylidene] hydrazone to N1-(tert-butoxycarbonyl)-N2-[4-(2'-pyridyl)benzyl]hydrazine;
   said process being **characterised in that** in step a) 4-(2'-pyridyl)benzaldehyde is provided by a process according to the present invention comprising a cross-coupling reaction between a halopyridine and an arylmagnesium halide in the presence of catalytic amounts of a zinc salt and of a palladium complex with a bidentate phosphine.

Both hydrazone formation in step b) and its reduction in step c) proceed in higher overall yields for the reagent which has the higher molar cost, i.e. the arylmagnesium halide, using 4-(2'-pyridyl)benzaldehyde provided according to the process of the present invention rather than according to the process disclosed in US 6,765,097 B1.

In addition the isolation of N1-(tert-butoxycarbonyl)-N2-[4-[(2-pyridylphenyl)]methylidene] hydrazone is easily accomplished, its reduction is faster and the final product N1-(tert-butoxycarbonyl)-N2-[4-(2'-pyridyl)benzyl]hydrazine is obtained with a better quality.

The invention will be now further illustrated by the following examples.

### Example 1

### 4-bromobenzaldehyde dimethyl acetal Grignard reagent

While regulating the temperature at 30-35°C, iodine (0.1 g) and then, over a period of approximately one hour, a solution of 4-bromobenzaldheyde dimethyl acetal (155.7 g, 0.674 mol) in tetrahydofuran (170 ml) are added to a suspension of magnesium (17.2 g, 0.708 mol) in tetrahydrofuran (290 ml) maintained at 30°C with stirring and under an inert atmosphere. The reaction mixture is maintained at 30°C for one hour.

### Exemple 2

### 4-(2'-pyridyl)benzaldheyde

Anhydrous zinc chloride (4.55 g, 33.5 mmol) and then 2-chloropyridine (80.3 g, 0.708 mol) are added, with stirring under inert atmosphere, in tetrahydrofuran (134 ml). (1,2-bis(diphenylphosphino)ethane)palladium(II) chloride (DPPE-palladium) (0.246 g, 0.43 mmol) and then over a period of two hours, the Grignard solution prepared analogously to Example 1, are added to the suspension maintained at 40°C with agitation and under inert atmosphere. The reaction is maintained at 40°C for about 30 minutes and then cooled to 25°C.

A solution of water (315 ml) and 37% hydrochloric acid (88 gr) is added to the reaction mixture over a period of approximately 30 minutes and then the solution is maintained under stirring for one hour. Toluene (130 ml) is added and the phases are separated. To the aqueous phase under stirring, toluene (350 ml) and then 30% ammonia solution (about 110 ml) are added. The phases are separated, the organic phase is evaporated under vacuum to yield a residue constituted by 4-(2'-pyridyl)benzaldehyde (118.4 g, 0.647 mol). The yield in moles relatives to the 4-bromobenzaldehyde dimethyl acetal is 96%. The turnover of the catalyst (DPPE-palladium) is 1504

The product was identified by comparison with an authentic sample prepared in accordance with Example 37b described in international patent application WO97/40029.

### Example 3

### 4-(2'-pyridyl)benzaldehyde

Anhydrous zinc chloride (4.1 g, 30 mmol) and then 2-chloropyridine (76.5 g, 0.674 mol) are added, with agitation under inert atmosphere, in tetrahydrofuran (135 ml). DPPE-palladium (0.228 g, 0.396 mmol) and then, over a period of 2 hours a solution of the Grignard reagent of 4-bromobenzaldheyde dimethyl acetal prepared analogously to Example 1, are added to the suspension maintained at 45°C with agitation and under inert atmosphere. After 30 minutes at 45°C the mixture is cooled to 25°C and a solution of water (300 ml) and 37% hydrochloric acid (83 g) is added over a period of approximately 30 minutes. Toluene (130 ml) is added and the phases are separated. Toluene (250 ml) and then 30% ammonia solution (105 ml) is added under stirring to the underlying aqueous phase. The phases are separated and the organic phase is titled by HPLC obtaining a content in 4-(2'-pyridyl)benzaldheyde of 117.3 g (0.640 mol) The yield in moles relative to the 4-bromobenzaldheyde dimethyl acetal is 95%. The turnover of the catalyst is 1616.

### Example 4

### N1-(tert-butoxycarbonyl)-N2-[4-[(2-pyridylphenyl)]methylidene] hydrazone;

To the toluene solution of 4-(2'-pyridyl)benzaldheyde of Example 3, acetic acid (2.7 ml) and then, at a temperature of 80°C under stirring, a solution of tert-butylcarbazate (89 g, 0.652 mol) in toluene (85 ml) are added. The mixture is maintained at 80°C for two hours and then cooled to 10°C. After 30 minutes the solid is filtered and washed with cold toluene. After drying at reduced pressure the hydrazone (184.3 g, 0.620 mol) is obtained. The molar yield relative to the 4-bromobenzaldheyde dimethyl acetal is 92%.

### Example 5

### N1-(tert-butoxycarbonyl)-N2-[4-(2-pyridyl)benzyl]hydrazine (comparative)

The procedure described in Example 28 of US 6,765,097 B1 was repeated.

5 g (0.0168 mol) of hydrazone of Example 4 and 0.5 g of palladium/C 5% (50% wet) in methanol (75 ml), are hydrogenated at ambient pressure for 8 hours. The catalyst is filtered and washed with methanol. The solved is removed by distillation at reduced pressure and to the oil residue is added cyclohexane. After stirring at ambient temperature for about one hour and at 15°C for 30 minutes, a solid is filtered and washed with cold cyclohexane. After drying at 40°C under reduced pressure, title hydrazine is obtained.
m.p. 77-79°C.
¹H-NMR (200MHz, CDCl₃): ppm 8.69 (1H, m); 7.69 (2H, d); 7.8-7.65 (2H, m); 7.22 (1H, m); 4.06 (2H, s); 1.47 (9H, s).

### Example 6

### N1-(t-butoxycarbohyl)-N2-(4-(2'pyridyl)behzyl)hydrazihe

50 g (0.168 mol) of hydrazone of Example 4, methanol (ml 350), ammonium formate (23.8 g, 0.378 mol), water (16 ml) and then Pd/C 50% wet (3.8 g) are charged in a flask and warm at 50°C over a period of about 3 hours. When the reaction is complete, the cold mixture is filtered and the solution concentrated to residue. Cyclohexane (200 ml) and then water (20 ml) are added and the mixture is warmed to 60°C. The organic phase is separated and cooled to 15°C for the crystallization of the product. After drying at 40°C under reduced pressure, hydrazine of the title (43.4 g, 0.145 mol) is obtained.

The molar yield relative to starting hydrazone is 86%.

The overall yield relative to the 4-bromobenzaldheyde dimethyl acetal is 79.2%.

### Example 7 (comparative)

The following table provides a comparison of the yields of the steps described in Examples 4 and 6 of the present invention, and of the overall yield of N1-(t-butoxycarbonyl)-N2-(4-(2'pyridyl)benzyl)hydrazine, wherein the starting 4-(2'-pyridyl)benzaldehyde is obtained according to the present invention (Entry #1), with the yields of the corresponding steps when the starting 4-(2'-pyridyl)benzaldehyde is obtained using the process described in US 6,765,097.

| **Entry #** | **Hydrazone % yield (based on Grignard reagent)** | **Reduction yield % (transfer hydrogenation)** | **Overall yield %** |
|---|---|---|---|
| 1 (aldehyde obtained according to the invention) | 92 | 86 | 79.2 |
| 2 (aldehyde obtained according to US 6,765,097) | 77.8 | 76 | 59.2 |

### Example 8

### Industrial preparation of N1-(t-butoxycarbohyl)-N2-(4-(2'pyridyl)benzyl)hydrazine

### Preparation of 4-bromobenzaldheyde dimethyl acetal Grignard reagent

In a suitable stainless steel vessel, under inert atmosphere, charge magnesium (49.7 kg), iodine (0.29 kg) and then THF (840 It). With agitation, charge 4-bromobenzaldheyde dimethyl acetal (40 kg) while the temperature is risen to 35-40°C. At this temperature attend the start of the reaction and then, add in 2-3 hours a solution of 4-bromo-benzaldheyde dimethyl acetal (kg 410) in THF (495 It).

Consider the reaction completed when the IPC by HPLC points out a content of 4-bromobenzaldheyde under 0.5%.

### Coupling reaction

In a stainless steel reactor, under inert atmosphere, charge THF (390 It), anhydrous zinc chloride (11.9 kg) and then 2-chloropyridine (222 kg). After 30 minutes add the DPPE-palladium (0.66 kg). Warm up the reaction at 35-40°C and add slowly the Grignard solution previously prepared. At the end keep the temperature at 35-40°C for about one hour. Consider the reaction completed when the HPLC control points up a content of benzaldheyde under 0.5%. Add slowly a solution of water (870 It) and hydrochloric acid 30% (about 290 kg). Charge toluene (380 It) and stir for 20 minutes, after that keep the mixture without stirring for one hour and then separate the phases. With agitation add toluene (It 380) and then slowly a 30% ammonia solution (about 300 It) to the underlying phase and stir for 30 minutes. Stop the stirring for one hour and then separate the phase. The organic phase is titled by HPLC obtaining a content in 4-(2'-pyridyl)benzaldheyde of about 340 kg.

### Preparation of N1-(tert-butoxycarbonyl)-N2-[4-[(2-pyridylphenyl)]methylidene] hydrazone

In a stainless steel reactor, under inert atmosphere, charge the organic phase of the previous step containing about 340 kg of 4-(2'-pyridyl)benzaldheyde and add acetic acid (7.8 It).Warm the solution at 80°C and add under stirring tert-butylcarbazate (258 kg). After two hours at 80°C cool the mixture at 15°C and then filter the product, wash with cold toluene and dry at reduced pressure at a temperature of 45°C obtaining about 540 kg of hydrazone.

Industrial reduction process for N1-(t-butoxycarbonyl)-N2-(4-(2'pyridyl)benzyl)hydrazine.

In a stainless steel reactor, under inert atmosphere, charge the hydazone (540 kg, 1.82 kmol), methanol (It 3200), ammonium formate (238 kg, 3.78 kmol), water (165 It) and then Pd/C 50% wet (38 kg).

Warm the mixture at 50°C under a good agitation. When the reaction is complete (residual hydrazone under 0.2% by HPLC test) cool the mixture at 25°C and filter the catalyst. Concentrate the filtrate solution under reduced pressure to obtain a residual viscous mass. Charge cyclohexane (1720 It) and water (167 It) and warm to 65°C. At this temperature separate the phase. Cool the organic phase at 25°C under stirring in order to have a complete crystallisation of the product.

Filter the product and wash the cake with cold cyclohexane. Dry at reduced pressure at a temperature of 45°C obtaining about kg 480 of N1-(t-butoxycarbonyl)-N2-(4-(2'pyridyl)benzyl)hydrazine.

## Claims

1. A process for the preparation of 4-(2'-pyridyl)benzaldehyde wherein:
(a) an arylmagnesium halide of formula 1 bis: 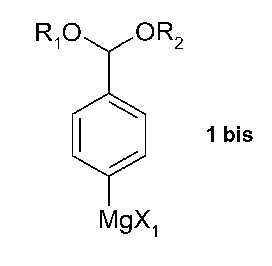 wherein X1 represents Cl, Br or I; R1 and R2 which are the same or different from one another, represent linear or branched C1-C6 alkyls or, alternatively, R1 and R2 together represent a single linear or branched C1-C6 alkylene group; is reacted with a halopyridine of formula 2 bis: 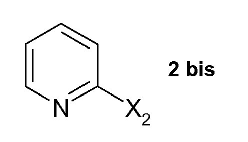 wherein X2 represents Cl, Br or I, in the presence of a catalytic amount of a zinc salt and of a catalytic amount a palladium complex with a bidentate phosphine, the molar ratio of said palladium complex to the halopyridine of formula 2 bis being less than 1:1000; and
(b) the intermediate compound so obtained is transformed into the desired compound by converting the acetal group into a carbonyl group.

2. A process according to claim 1, **characterized in that** the arylmagnesium halide of formula 1 bis is used in dynamic deficiency relative to the zinc salt.

3. A process according to claim 1, **characterized in that** the halopyridine of formula 2 bis is 2-chloropyridine.

4. A process according to claim 1, **characterized in that** the arylmagnesium halide of formula 1 bis is a bromide or a chloride.

5. A process according to claim 1, **characterized in that** the zinc salt is selected from ZnCl₂, ZnBr₂ and/or Zn(OAc)₂.

6. A process according to claim 1, **characterized in that** the zinc salt is present in an amount of 1 - 50 moles, preferably 4 - 30 moles, per 100 moles of halopyridine of formula 2 bis.

7. A process according to claim 1, **characterized in that** the palladium complex with a bidentate phosphine is selected from the group of (1,2-Bis(diphenylphosphino)ethane)palladium(II) chloride, (1,3-Bis(diphenylphosphino)propane) palladium(II) chloride and (1,4-Bis(diphenylphosphino)butane)palladium(II) chloride.

8. A process according to claim 7, **characterized in that** the palladium complex with a bidentate phosphine is (1,2-Bis(diphenylphosphino)ethane)palladium(II) chloride.

9. A process according to claim 1, **characterized in that** the palladium complex with a bidentate phosphine is used in an amount of 0.01 - 1 mole, preferably 0.05 - 0.1 mole, per 100 moles of halopyridine of formula 2 bis.

10. A process according to claim 1, **characterized in that** the halopyridine of formula 2 bis is used in an amount of 0.8 -1.2 moles, per mole of arylmagnesium halide of formula 1 bis.

11. A process according to claim 1, **characterized in that** stage (a) is carried out at a temperature of 0 - 85°C, preferably at 30 - 50°C.

12. A process according to claim 1, **characterized in that** stage (a) is carried out in an aprotic organic solvent, preferably in tetrahydrofuran and/or in toluene.

13. A process according to claim 1, **characterized in that** stage (b) is carried out by acid hydrolysis.

14. A process according to claim 13, **characterized in that** the acid hydrolysis is carried out at temperatures lower than 40°C.

15. A process according to claim 1, **characterized in that** R1 and R2 are both methyl.

16. A process according to claim 1, **characterized in that** R1 and R2, taken together, are selected from 1,3-propyl, 1,2-butyl, 1,4-butenyl and 2,2-dimethyl-1,3-propyl.

17. A process according to claim 1, **characterized in that** the molar ratio of the palladium complex with a bidentate phosphine to the halopyridine of formula 2 bis is from 1:3000 to 1:1000.

18. A process for the preparation of an azahexane heterocyclic derivative having antiviral action of formula 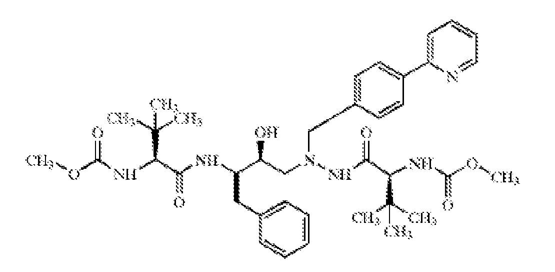 **characterized in that** it comprises a process according to claims 1-17.

19. A process for the preparation of N-1-(tert-butoxycarbonyl)-N-2-[4-(2-pyridyl)-benzyl]-hydrazine or N-1-(tert-butoxycarbonyl)-N-2-{4-[(2-pyridyl)-phenyl]methylidene}-hydrazone, **characterized in that** it comprises a process according to claims 1-17.
